# EUROPEAN PATENT SPECIFICATION

(11) **EP 4 403 125 B1**
(45) Date of publication and mention of the grant of the patent: **24.09.2025**
(21) Application number: 23305061.6
(22) Date of filing: 18.01.2023
(51) Int. Cl.: A61N 5/10, A61B 90/50, A61N 7/02, G06T 15/08, G06T 15/20, G06T 17/20, A61B 18/20, A61B 34/10, A61B 18/04, G06T 19/00, G16H 30/20, G16H 30/40, G16H 50/50, G16H 20/40

(54) **COMPUTER-IMPLEMENTED METHOD FOR MEDICAL GUIDANCE OF A USER IN THE VALIDATION OF A TREATMENT DOSE DISTRIBUTION PROVIDED BY A TREATMENT PLANNING SYSTEM**
COMPUTERIMPLEMENTIERTES VERFAHREN ZUR MEDIZINISCHEN FÜHRUNG EINES BENUTZERS BEI DER VALIDIERUNG EINER VON EINEM BEHANDLUNGSPLANUNGSSYSTEM BEREITGESTELLTEN BEHANDLUNGSDOSISVERTEILUNG
PROCÉDÉ MIS EN OEUVRE PAR ORDINATEUR POUR LE GUIDAGE MÉDICAL D'UN UTILISATEUR DANS LA VALIDATION D'UNE DISTRIBUTION DE DOSE DE TRAITEMENT FOURNIE PAR UN SYSTÈME DE PLANIFICATION DE TRAITEMENT

(43) Date of publication of application: 24.07.2024
(73) Proprietor: Avatar Medical, 75015 Paris (FR)
(72) Inventor: EL BEHEIRY, Mohamed, 75015 Paris (FR); BRIENT-LITZLER, Elodie, 75915 Paris (FR)
(74) Representative: Icosa

(56) References cited:
- JP-A- 2019 130 392
- US-A1- 2006 239 538
- US-A1- 2020 054 398
- US-B1- 6 815 687

## Description

### FIELD OF INVENTION

The present invention relates to a computer-implemented method for medical guidance of a user in the validation of a treatment dose distribution provided by a treatment planning system.

### BACKGROUND OF INVENTION

It is known to use a series of images of a subject's body area taken before an intervention. This series of images, called "planning data" or "planning images", is used by doctors to prepare the intervention, in particular by visualizing the body area on which they must intervene and by locating the diseased tissues as well as determine areas and tissues that are to be preserved. Typically, a three-dimensional model of the body area is computed from the planning images.

In the case of medical interventions where a treatment has to be delivered to a target region of a body subject by the doctors via a probe, precise knowledge of how the probe will interact with the target region is needed, so as to maximize the effects of the treatment while minimizing the risks this treatment may bring.

In the context of radiotherapy or ablation therapy, theoretical three-dimensional treatment dose distributions can be obtained from a treatment planning system. The theoretical three-dimensional treatment dose distributions can be superimposed on a three-dimensional model of the targeted body area computed from planning images. For example, document US 2020/054398 discloses a met hod wherein a cloud of points associated with treatment plan data is generated and overlaid over a model of the patient to be treated.

During the planning phase, it is of help to manipulate a theoretical three-dimensional treatment dose distribution so as to visualize how it will interact with the targeted body area.

However, the current user interfaces available for doctors only display two-dimensional slices, which are difficult to analyze, difficult to manipulate and lack efficient and comfortable visualization properties for the doctors to get intuitive insight on how the theoretical three-dimensional treatment dose distribution will interact with the target body area.

The invention aims at improving the situation.

### SUMMARY

This invention thus relates to a computer-implemented method for medical guidance of a user in the validation of a treatment dose distribution provided by a treatment planning system, comprising:
- receiving a body 3D model of at least a portion of a body of a subject,
- receiving treatment plan data representative of a 3D distribution of a treatment dose, to be delivered to a region of the body of the subject during a radiotherapy, an ablation, a laser-based or an ultrasound-based treatment,
- generating a cloud of points based on at least a subset of said treatment plan data, wherein each point is associated with a landmark having displaying characteristics comprising at least a shape, a size, an intensity, a transparency, an orientation, said displaying characteristics being associated with corresponding treatment plan data,
- displaying a three-dimensional scene comprising an overlay of the body 3D model and said cloud of points,
wherein the displaying characteristics of the landmarks associated to said treatment plan data in different depth planes of the three-dimensional scene are distinct, so that said landmarks are better perceived in comparison to classical dose representation of continuous colored overlay.

This allows better visualizing treatment plan data corresponding to different depth planes in the three-dimensional scene, and as a consequence, better visualizing the localization of the treatment plan data. Indeed, the inventors have found that the visibility of an object underneath a cloud of points is better than underneath a continuous colored layer. Specifically, the inventors have found that the difference in depth of two landmarks associated with different depths in the three-dimensional scene is better visualized with landmarks associated with points of a point cloud than with a continuous colored layer

In some embodiments, the step of generating the cloud of points comprises:
- a) creating a mesh from said treatment plan data, said mesh comprises a plurality of vertices, and wherein, for at least a subset of vertices, each vertex corresponds to a point of the cloud of points.

In some embodiments, the mesh has a highly symmetrical pattern. For instance, when the treatment plan data is a 3D image composed of a plurality of voxels, the mesh is a cubic crystal system wherein each vertex corresponds to a voxel in the 3D image.

In some embodiments, the step of generating the cloud of points further comprises:,
b) applying a random coordinate offset to each vertex, said random coordinate offset being smaller than a distance between two neighboring vertices, to obtain offset points, and
c) associating to each offset point a landmark,
wherein the step of displaying the three-dimensional scene comprises displaying the landmarks at coordinates of the offset points.

Therefore, when the representation of the cloud of points is displayed overlaid on the three-dimensional scene on the display unit, the representation of the cloud of points comprises the offset points. Therefore, the offset points are not aligned as would be points coincident with vertices of the highly symmetrical mesh created from the treatment plan data at step a), but are slightly offset and thus are better visible by a user due to the random offsets.

In an embodiment, one of the displaying characteristics is representative of a dose intensity associated with the treatment plan data.

In some embodiments, for at least one subset of the landmarks, the intensity is variable in time with a predetermined frequency, so as to ease the visualization of said anatomic structures representation belonging to the body 3D model, notably in the region of the body of the subject corresponding to the distribution of the treatment dose.

In some embodiments, the method further comprises, before generating said cloud of points, receiving a point density parameter, and wherein the step of generating the cloud of points is based on said point density parameter.

In some embodiments, the cloud of points is dynamic and at least one subset of the landmarks is moving in the vicinity of its initial coordinates, so as to ease the visualization of said anatomic structures representation belonging to the body 3D model, notably in the region of the body of the subject corresponding to the distribution of the treatment dose.

In some embodiments, the step of displaying the three-dimensional scene is implemented on a stereoscopic display unit, so as to further increase the perception of the treatment plan data in the 3D space with an enhanced perception of depth.

In some embodiments, the method further comprises receiving at least one interest 3D model of a structure of interest of the subject, and wherein for a first subset of the landmarks, at least one displaying characteristic is assigned to a first value, and for a second subset of the landmarks, said at least one displaying characteristic is assigned to a second value, said second value being different from the first value, wherein the first subset of landmarks is located inside of the at least one interest 3D model, and the second subset of landmarks is located outside of the interest 3D model.

In some embodiments, the structure of interest is representative of an organ at risk.

In some other embodiments, the structure of interest is a target zone.

For instance, target zone is representative of at least one among gross tumor volume, a clinical target volume, or a planning target volume.

In some embodiments, for each point in the cloud of points, at least one among the displaying characteristics of the corresponding landmark is defined based on a number of beams used to produce the corresponding dose treatment data associated to the point and/or a number of probes to produce the treatment plan data.

In some embodiments, the displaying characteristics associated with corresponding treatment plan data are associated with displaying data being defined as a decreasing function of said 3D distribution treatment dose. In an embodiment, the displaying characteristics are associated to a decreasing function (e.g. inverse function) of the dose intensity and enables the user to identify zones that have not received the treatment.

In some embodiments, the method further comprises the steps of:
- receiving second treatment plan data representative of a second 3D distribution of a second treatment dose, to be delivered to said region of the body of the subject during a radiotherapy or an ablation or a laser-based or an ultrasound-based treatment,
- generating a second cloud of points based on said second treatment plan data, wherein each point of the second cloud of points is associated with a landmark having displaying characteristics comprising at least a shape, a size, and an intensity, said displaying characteristics being associated with corresponding second treatment plan data,
- displaying an overlay of the three-dimensional scene and said second cloud of points.

In some embodiments, the body 3D model is obtained based on a plurality of images comprising said at least portion of a body of the subject, said images being CT scan images, MRI images, or cone beam CT images.

In some embodiments, the method further comprises the steps of:
- computing at least one 2D projection of said three-dimensional scene on at least one 2D projection plane;
- displaying said at least one 2D projection.

In some embodiments, the treatment plan data include information about a type of technology, a type of device, a number of devices, a number of beams, or parameters used in device settings to obtain said treatment plan data.

In some embodiments, a subset of the treatment plan data can be selected through a user interface and the three-dimensional scene displayed in the displaying step comprises an overlay of the body 3D model and of landmarks corresponding to the subset of the treatment plan data.

### BRIEF DESCRIPTION OF THE DRAWINGS

Figure 1 is a schematic representation of a device configured to implement the method according to embodiments of the present invention.
Figure 2 is a schematic representation of a processing unit of the device of Figure 1.
Figure 3 is an example of flow chart of the method for medical guidance of a user in the validation of a treatment dose distribution provided by a treatment planning system.
**Figure 4** shows different three-dimensional scenes with an overlay of a body 3D model with a cloud of points, for different points size.
**Figure 5** represents different superpositions of a body 3D model with a cloud of points for different point density values.
**Figure 6** shows an overlay of a cloud of points representative of treatment plan data and segmented organs at risk.
**Figure 7** shows a series of radiographic images with cloud of points representing treatment dose data and corresponding to different threshold conditions.
**Figure 8** shows a comparative of two clouds of points, on the left without an offset of the vertices coordinates and on the right with a random offset of the vertices coordinates.

### DETAILED DESCRIPTION

The term "processing unit" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

It is assumed here that a subject is about to undergo a medical intervention. During this intervention, a portion of his body will be treated by doctors via at least one probe or via at least a beam. For instance, in the case of a radiotherapy intervention, a beam will illuminate the portion of the subject's body. In the case of an ablation therapy, the probe will deliver energy to heat or cool the portion of the subject's body. In the case of an ultrasound-based treatment, ultrasound waves will impinge on the portion of the subject's body.

The invention relates to a computer-implemented method for medical guidance of a user in the validation of a treatment dose distribution provided by a treatment planning system.

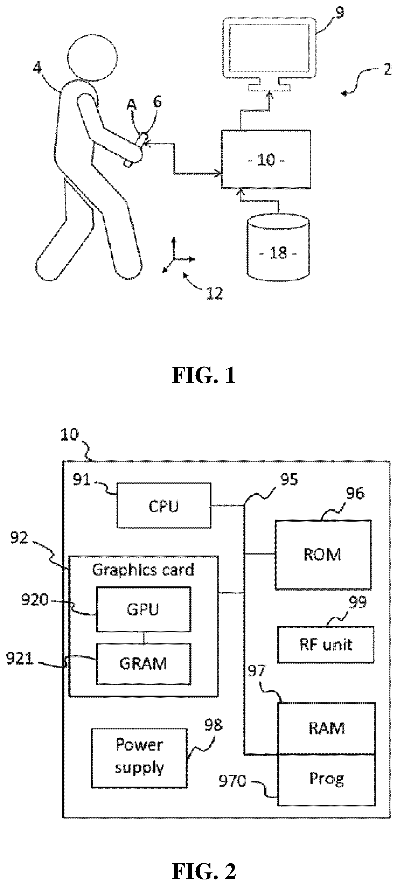

This method may be implemented by a device as illustrated in Figure 1.

Figure 1 is a schematic representation of a device configured to implement embodiments of a method for medical guidance of a user in the validation of a treatment dose distribution provided by a treatment planning system.

The device is advantageously an apparatus, or a physical part of an apparatus, designed, configured and/or adapted for performing the mentioned functions and produce the mentioned effects or results. In alternative implementations, the device is embodied as a set of apparatus or physical parts of apparatus, whether grouped in a same machine or in different, possibly remote, machines. The device may for example have functions distributed over a cloud infrastructure and be available to users as a cloud-based service, or have remote functions accessible through an API ("application programming interface").

The device includes a user graphical interface 2, comprising a controller 6 for interacting with a three-dimensional scene, that will be described more in details below, a display unit 9 for displaying the three-dimensional scene, and a processing unit 10 for processing data received from the controller 6 and for controlling the display unit 9.

### Controller 6

The controller 6 is configured to acquire a current position of a predetermined user point A, of a user 4, in a predetermined user coordinate system 12. The user 4 can be a doctor or any other health professional.

The predetermined user coordinate system 12 is, for instance, fixed with respect to the user's environment. The predetermined user coordinate system 12 uses one or more numbers, or coordinates, to uniquely determine the position of points or other geometric elements in the user's environment.

The controller 6 may include a handheld motion tracking sensor, thereby allowing the user 4 to interact with the three-dimensional scene using hand gestures. In this case, the user point A may be a point of the motion tracking sensor.

The controller 6 may also include other devices, such as a touch screen, a game controller, a mouse and/or a keyboard, thereby further allowing the user 4 to interact with the three-dimensional scene as will be explained later.

The controller 6 may further include buttons and/or switches configured to allow the user 4 to interact with the three-dimensional scene. Such buttons and/or switches may be included in the handheld motion tracking sensor, or may be included in a separate device of the controller 6, such as the touch screen, game controller, mouse and/or keyboard mentioned above.

The controller 6 may include a 3D controller connected to a virtual reality headset.

Preferably, the controller 6 is configured to allow the user 4 to input (for example through said buttons and/or switches) specific instructions, such as an object display instruction, an object shifting instruction or a transformation instruction. Such instructions advantageously allow the user 4 to add and/or manipulate 3D objects in the three-dimensional scene, as will be described below.

The controller 6 may also be configured to allow the user to manipulate *(e.g.,* to rotate and/or to zoom in on or out of) 3D objects in the three-dimensional scene, and/or to change a direction along which the user 4 views the three-dimensional scene displayed by the display unit 9.

Alternatively, or in addition, the controller 6 includes virtual buttons displayed in the three-dimensional scene to allow the user 4 to interact with the three-dimensional scene, and 3D objects in the three-dimensional scene.

### Display unit 9

As mentioned previously, the display unit 9 is configured to display the three-dimensional scene, that will be described below.

The display unit 9 may include a screen, as shown on figure 1.

Alternatively, and advantageously, the display unit 9 is at least part of a virtual reality headset, thereby allowing stereoscopic visualization of the three-dimensional scene. This is particularly advantageous in the field of medicine, since virtual reality visualization allows the user 4 to have a good understanding of actual volumes and precise localization of objects of interest.

### Processing unit 10

The processing unit 10 is connected to each of the controller 6 and the display unit 9.

The processing unit 10 corresponds, for example, to a workstation, a laptop, a tablet, a smartphone, programmable logical device (e.g., FPGA) for on-board calculation or a head-mounted display (HMD) such as a virtual reality headset.

As shown on figure 2, the processing unit 10 may comprise the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing due to their highly parallel structure;
- a non-volatile memory of ROM type 96;
- a RAM 97;
- a power supply 98; and
- a radiofrequency unit 99.

Alternatively, the power supply 98 is external to the processing unit 10.

The controller 6 is, for instance, connected to at least part of the aforementioned modules, for instance through the bus 95.

The display unit 9 is connected to the graphics card 92, for instance through a suitable interface. For instance, a cable can be used for tethered transmissions, or the RF unit 99 can be used for wireless transmissions.

Each of memories 97 and 921 includes registers, which can designate in each of said memories, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner. Each of them does not necessarily correspond to adjacent memory locations and can be distributed otherwise (which covers notably the situation in which one register includes several smaller registers).

When switched-on, the microprocessor 91 loads and executes the instructions of the program 970 contained in the RAM 97 to allow operation of the visualization device 2 in the fashion described in the present disclosure.

As will be understood by a skilled person, the presence of the graphics card 92 is not mandatory, and can be replaced with entire CPU processing and/or other implementations.

### Operation

The device is configured to receive as input 18 a three-dimensional model 3 of the portion of the subject's body. This three-dimensional model 3 is referred to in what follows as body 3D model 3.

The body 3D model 3 can have been preliminary computed based on 2D images of the portion of the subject's body, such as CT scan ("Computed Tomography scan") images, PET ("Positron Emission Tomography") images, MRI ("Magnetic Resonance Imaging") images, or cone beam CT images. Alternatively, the device may be configured to receive as input 18 2D images of the portion of the subject's body, and then compute via the processing unit 10, from the received 2D images, the body 3D model.

The display unit 9 of the user graphical interface 2 is configured to display, to the user 4, a three-dimensional scene comprising the body 3D model 3.

The device is configured to further receive as input 18 treatment plan data representative of a 3D distribution of a treatment dose, to be delivered to a region of the body of the subject during a radiotherapy or an ablation therapy or an ultrasound-based treatment.

The processing unit 10 is configured, after reception of the treatment plan data, to generate a cloud of points based on the treatment plan data. Each point in the cloud of point is associated with a landmark having displaying characteristics. Examples of displaying characteristics comprise a shape, a size, an intensity, and a transparency. The displaying characteristics are associated with the corresponding treatment plan data.
In some embodiments, after a cloud of points has been generated, a representation of the cloud of points is displayed overlaid on the three-dimensional scene on the display unit 9. In particular, the representation of the cloud of points is overlaid on the body 3D model 3. This allows better visualizing treatment plan data corresponding to different depth planes in the three-dimensional scene, and as a consequence, better visualizing the localization of the treatment plan data. Indeed, the inventors have found that the visibility of an object underneath a cloud of points is better than underneath a continuous colored layer. In addition, the difference in depth of two landmarks associated with different depths in the three-dimensional scene is better visualized with landmarks associated with points of a point cloud than with a continuous colored layer. Therefore, the use of a cloud of points to visualize the body parts underneath this cloud of points helps a user visualizing in a faster manner those body parts. In that respect, the use of a cloud of points to represent the 3D distribution of a treatment dose contributes to the technical effect of faster visualization of the body parts underneath the cloud of points and of the positioning of treatment plan data over those body parts.

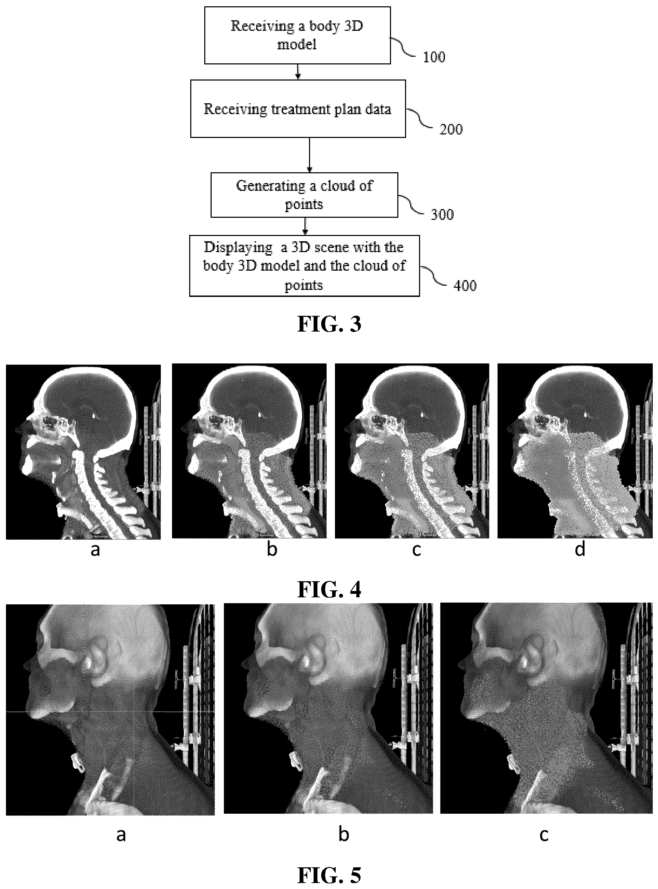

Figure 4 shows a three-dimensional scene with an overlay of a body 3D model with a cloud of points, for different points sizes. From Figure 4a to figure 4d, the point size increases. Therefore, a user can tailor the display of the three-dimensional scene at wish.

Advantageously, the processing unit 10 is configured to generate the cloud of points by:
a) creating a mesh from said treatment plan data, said mesh comprises a plurality of vertices, and wherein each vertex corresponds to a point of the cloud of points,
b) applying a random coordinate offset to each vertex, said random coordinate offset being smaller than a distance between two neighboring vertices, to obtain offset points,
c) associating to each offset point a landmark.

Therefore, when the representation of the cloud of points is displayed overlaid on the three-dimensional scene on the display unit 9, the representation of the cloud of points comprises the offset points. Therefore, the offset points are not aligned as would be points coincident with vertices of a highly symmetrical mesh created from the treatment plan data at step a), but are slight offset and thus are better visible by a user.

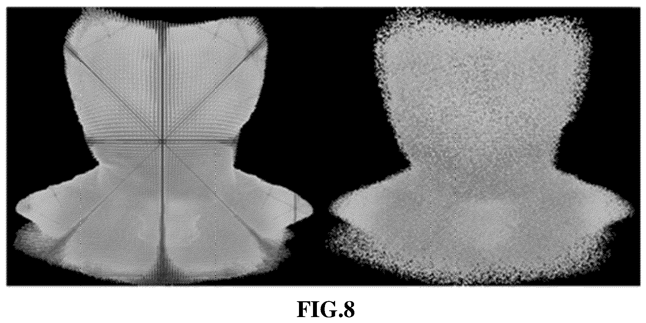

For example, Figure 8 on the left shows a first cloud of points wherein the points are coincident with vertices of an initial cubical symmetrical mesh, and Figure 8 on the right shows a second cloud of points wherein the vertices and corresponding points are randomly offset with respect to the initial mesh. Without the offset, one can see artifacts and less accuracy to distinguish between different colors at different depths of the 3D image.

In some embodiments, for at least one subset of the landmarks of the cloud of points, the intensity of the landmarks is not still, and, on the contrary, blinks. In other words, the intensity of the at least one subset of landmarks is variable in time with a predetermined frequency. This further eases the visualization of, for instance, the representation of anatomical structures belonging to the body 3D model and lying underneath the at least one subset of the landmarks.

In some embodiments the display of the cloud of points is dynamic (i.e. not static) and at least one subset of the landmarks is moving (i.e. is displaced) in the vicinity of its initial coordinates, so as to ease the visualization of said anatomic structures representation belonging to the body 3D model, notably in the region of the body of the subject corresponding to the distribution of the treatment dose. The landmark can for instance move (e.g. periodically) around a circle (or other closed trajectories) centered on the coordinates of the vertex it originates from.

In some embodiments, it is possible to set a point density for the cloud of points, before generating it. Varying the point density of the cloud of points allows for customization of the user's visualization settings. In this manner, the user 4 can adjust the point density to his or her convenience.

Figure 5 illustrates a superposition of a body 3D model and a cloud of points for different point density values. From Figure 5a to Figure 5c, the point density parameter increases. Therefore, more points are visible on Figure 5c than on Figure 5a.

In some embodiments, the device is configured to receive at least one 3D model of a structure of interest of the subject, referred to as interest 3D model.

For instance, the structure of interest of the subject is one among a gross tumor volume, a clinical target volume, or a planning target volume. The structure of interest is then referred to as a target zone that has to be reached by the 3D distribution of the treatment dose.

In another example, the structure of interest of the subject is an organ at risk, that has to be avoided by the treatment 3D distribution.

Therefore, in some embodiments where the device receives at least one interest 3D model of a structure of interest of the subject, the cloud of points is divided into two subsets, for instance into a first subset and a second subset. In a first subset of the landmarks located inside of the at least one interest 3D model, at least one displaying characteristic is assigned to a first value. In a second subset of the landmarks located outside of the interest 3D model, said at least one displaying characteristic is assigned to a second value different from the first value. This difference in displaying characteristics allows distinguishing the points inside the structure of interest of the subject from the points outside the structure of interest of the subject. If the structure of interest is one among a gross tumor volume, a clinical target volume, or planning target volume, in this manner, the user 4 can better visualize the effect of the treatment dose inside the structure of interest, and assess if there is a need to optimize this effect. If the structure of interest is an organ at risk, the user 4 can better visualize whether the treatment dose affects the organ at risk, and reacts, for instance to modify the treatment dose.

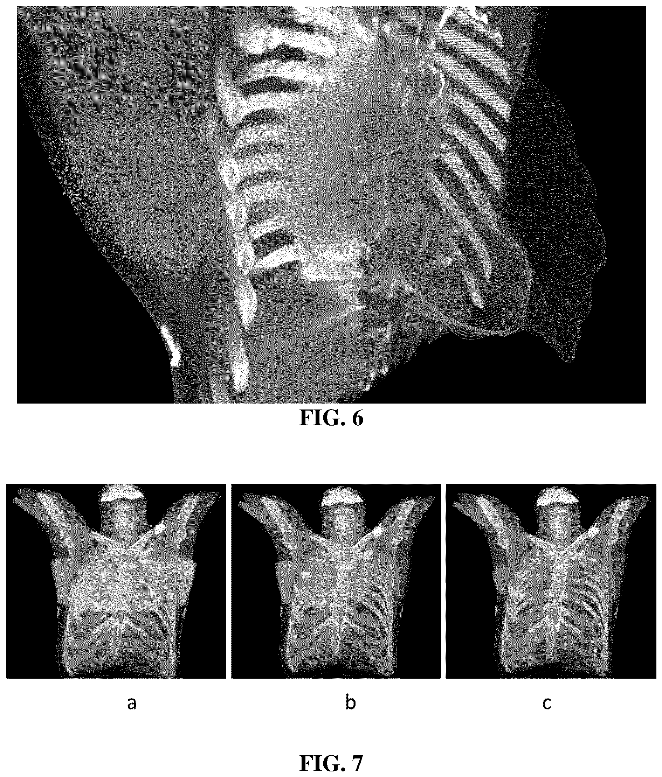

Figure 6 shows an overlay of a cloud of points representative of treatment plan data and segmented organs at risk.

Advantageously, the treatment plan data are associated with a specific position and orientation of a virtual probe representative of a real probe that would deliver the treatment plan data in real life. Alternatively, the treatment plan data are associated with a specific orientation of a virtual beam representative of a real beam that would deliver the treatment plan data in real life. In this case, it may be possible to vary the position and orientation of the virtual probe, or the virtual beam, with the controller 6. In other words, when the controller 6 is moved in space, in the user's environment comprising the predetermined user coordinate system 12, the position and orientation of the virtual probe is varied accordingly, therefore modifying corresponding coordinates of the treatment plan data with respect to the body 3D model.

Sometimes, the treatment plan data may have been obtained with a plurality of virtual probes (or virtual beams). This is the case for instance when one unique probe, or one unique beam, was not sufficient to deliver enough treatment to a targeted region of the subject's body.

Therefore, in some embodiments, for each point in the cloud of points, at least one among the displaying characteristics of the corresponding landmark is defined based on a number of beams used to produce the corresponding dose treatment data associated to the point and/or a number of probes to produce the treatment plan data. Visualizing the number of beams or probes producing the treatment dose data at a given location of the subject's body allows for adjustment, by the user 4, of the number of beams, or probes, to achieve a given overall treatment dose distribution.

In other embodiments, separate treatment dose data corresponding to separate probes or beams may be received by the device. For instance, the device is configured to receive, in addition to initial treatment dose data, second treatment plan data representative of a second 3D distribution of a second treatment dose, to be delivered to the targeted region of the body of the subject during the medical intervention. In this case, the method further comprises:
- generating a second cloud of points based on the second treatment plan data, wherein each point of the second cloud of points is associated with a landmark having displaying characteristics comprising at least a shape, a size, and an intensity, said displaying characteristics being associated with corresponding second treatment plan data,
- displaying in overlay of the three-dimensional scene the second cloud of points. In those embodiments, the user can visualize the overall effect of all separate treatment doses and assess the efficiency of this overall effect.

In some embodiments, displaying characteristics associated with corresponding treatment plan data are associated with displaying data being defined as a decreasing function of said 3D distribution treatment dose. For instance, the intensity of the landmarks is a decreasing function of the 3D distribution treatment dose. This helps enhancing the locations in the body 3D model where the treatment dose would not be sufficient and would provoke a reaction of the user 4. Such a reaction could be the manipulation of the controller 6 to displace the cloud of points, or a new computation of treatment dose data with an additional probe or beam.

In some embodiments, the processing unit 10 is configured to compute at least one 2D projection of said three-dimensional scene on at least one 2D projection plane. In this case, the displaying unit is further configured to display at least one 2D projection.

In some embodiments, the treatment plan data include information about a type of technology, a type of device, or parameters used in device settings to obtain said treatment plan data. For instance, the treatment plan data can indicate whether the treatment is an X-ray treatment, a treatment delivered by an electron beam, or by a proton beam. The treatment plan data can also indicate the energy level of the beam used for the treatment. Therefore, one displaying characteristic may pertain to one among such information.

In some embodiments, a subset of the treatment plan data can be selected through a user interface and wherein the three-dimensional scene displayed in the displaying step comprises an overlay of the body 3D model and of landmarks corresponding to the subset of the treatment plan data. This allows for example applying a threshold condition to the treatment plan data and displaying the landmarks corresponding to the treatment plan data satisfying the threshold condition.

Figure 7 shows a series of radiographic images with cloud of points corresponding to different threshold conditions (from Figure 7 a to Figure 7 c, the threshold condition corresponds to a higher dose intensity value. Therefore, less points are visible on Figure 7 c in contrast to Figure 7 a). For instance, a threshold condition can be input via a user interface by a user. As a consequence, only the points in the cloud of points satisfying the threshold condition can be displayed.

## Claims

1. A computer-implemented method for medical guidance of a user in the validation of a treatment dose distribution provided by a treatment planning system, comprising:
- receiving (100) a body 3D model of at least a portion of a body of a subject,
- receiving (200) treatment plan data representative of a 3D distribution of a treatment dose, to be delivered to a region of the body of the subject during a radiotherapy, an ablation, a laser-based or an ultrasound-based treatment,
- generating (300) a cloud of points based on at least a subset of said treatment plan data, wherein each point is associated with a landmark having displaying characteristics comprising at least a shape, a size, an intensity, a transparency, or an orientation, said displaying characteristics being associated with corresponding treatment plan data,
- displaying (400) a three-dimensional scene comprising an overlay of the body 3D model and said cloud of points,
wherein the displaying characteristics of the landmarks associated to said treatment plan data in different depth planes of the three-dimensional scene are distinct, so that said landmarks are better perceived in comparison to classical dose representation of continuous colored overlays.

2. The method according to claim 1, wherein the step of generating (300) the cloud of points comprises:
a) creating a mesh from said treatment plan data, said mesh comprises a plurality of vertices, and wherein, for at least a subset of vertices, each vertex corresponds to a point of the cloud of points.

3. The method according to claim 2, further comprising the steps of:
b) applying a random coordinate offset to each vertex, said random coordinate offset being smaller than a distance between two neighboring vertices, to obtain offset points, and
c) associating to each offset point a landmark,
wherein the step of displaying (400) the three-dimensional scene comprises displaying the landmarks at coordinates of the offset points.

4. The method according to any one of the preceding claims, wherein one of the displaying characteristics is representative of a dose intensity associated with the treatment plan data.

5. The method according to any one of the preceding claims, wherein for at least one subset of the landmarks, the intensity is variable in time with a predetermined frequency, so as to ease the visualization of said anatomic structures representation belonging to the body 3D model, notably in the region of the body of the subject corresponding to the distribution of the treatment dose.

6. The method according to any one of the preceding claims, further comprising, before generating said cloud of points, receiving a point density parameter, and wherein the step of generating the cloud of points is based on said point density parameter.

7. The method according to any one of the preceding claims, wherein the cloud of points is dynamic and at least one subset of the landmarks is moving in the vicinity of its initial coordinates, so as to ease the visualization of said anatomic structures representation belonging to the body 3D model, notably in the region of the body of the subject corresponding to the distribution of the treatment dose.

8. The method according to any one of the preceding claims, wherein the step of displaying the three-dimensional scene is implemented on a stereoscopic display unit, so as to further increase the perception of the treatment plan data in the 3D space with an enhanced perception of depth.

9. The method according to any one of the preceding claims, further comprising receiving at least one interest 3D model of a structure of interest of the subject, and wherein for a first subset of the landmarks, at least one displaying characteristic is assigned to a first value, and for a second subset of the landmarks, said at least one displaying characteristic is assigned to a second value, said second value being different from the first value, wherein the first subset of landmarks is located inside of the at least one interest 3D model, and the second subset of landmarks is located outside of the interest 3D model.

10. The method according to claim **9,** wherein the structure of interest is representative of an organ at risk.

11. The method according to claim **9,** wherein the structure of interest is a target zone, preferably the target zone is representative of at least one among gross tumor volume, a clinical target volume, or a planning target volume.

12. The method according to any of the preceding claims, further comprising the steps of:
- receiving second treatment plan data representative of a second 3D distribution of a second treatment dose, to be delivered to said region of the body of the subject during a radiotherapy or an ablation or a laser-based or an ultrasound-based treatment,
- generating a second cloud of points based on said second treatment plan data, wherein each point of the second cloud of points is associated with a landmark having displaying characteristics comprising at least a shape, a size, and an intensity, said displaying characteristics being associated with corresponding second treatment plan data,
- displaying an overlay of the three-dimensional scene and said second cloud of points.

13. The method according to any of the preceding claims, wherein said body 3D model is obtained based on a plurality of images comprising said at least portion of a body of the subject, said images being CT scan images, MRI images, or cone beam CT images.

14. The method according to any of the preceding claims, further comprising the steps of:
- computing at least one 2D projection of said three-dimensional scene on at least one 2D projection plane;
- displaying said at least one 2D projection.

15. The method according to any of the preceding claims, wherein the treatment plan data include information about a type of technology, a type of device, a number of devices, a number of beams, or parameters used in device settings to obtain said treatment plan data.

## Patentansprüche

1. Computerimplementiertes verfahren zur medizinischen führung eines benutzers bei der validierung einer von einem behandlungsplanungssystem bereitgestellten behandlungsdosisverteilung, bestehend aus:
- Empfangen (100) eines 3D-Körpermodells zumindest eines Körperabschnitts eines Subjekts,
- Empfangen (200) von Behandlungsplandaten, die eine 3D-Verteilung einer Behandlungsdosis repräsentieren, welche während einer Strahlentherapie, einer Ablation, einer laserbasierten oder einer ultraschallbasierten Behandlung in einem Bereich des Körpers des Subjekts verabreicht werden soll,
- Erzeugen (300) einer Punktwolke auf der Grundlage zumindest eines Teilbereichs der genannten Behandlungsplandaten, wobei jedem Punkt ein Landmarke zugeordnet ist, die Anzeigecharakteristika aufweist, welche mindestens eine Form, eine Größe, eine Intensität, eine Transparenz oder eine Ausrichtung umfassen, wobei die o.g. Anzeigecharakteristika den entsprechenden Behandlungsplandaten zugeordnet sind,
- Anzeigen (400) einer dreidimensionalen Szene, die eine Überlagerung des 3D-Körpermodells und der o.g. Punktwolke umfasst,
wobei die Anzeigecharakteristika der mit den genannten Behandlungsplandaten in unterschiedlichen Tiefenebenen der dreidimensionalen Szene verknüpften Landmarken deutlich unterscheidbar sind, sodass die genannten Landmarken im Vergleich zu einer klassischen Dosisdarstellung durch kontinuierliche farbige Überlagerungen besser wahrnehmbar sind.

2. Der Verfahren nach Anspruch 1, wobei der Schritt des Erzeugens (300) der Punktwolke umfasst:
a) Erzeugen eines Mesh-Modells aus den genannten Behandlungsplandaten, wobei das Modell eine Vielzahl von Vertizes umfasst und wobei für zumindest einen Teil der Vertizes jeder Vertex einem Punkt der Punktwolke entspricht.

3. Der Verfahren nach Anspruch 2, ferner umfassend die Schritte:
b) Anwenden eines zufälligen Koordinatenoffsets auf jeden Vertex, wobei der zufällige Koordinatenoffset kleiner ist als der Abstand zwischen zwei benachbarten Vertizes, um sog. Offsetpunkte zu erhalten, und
c) Zuordnen einer Landmarke zu jedem Offsetpunkt, wobei der Schritt des Anzeigens (400) der dreidimensionalen Szene das Anzeigen der Landmarken an den Koordinaten der Offsetpunkte umfasst.

4. Der Verfahren nach einem der vorhergehenden Ansprüche, wobei eines der Anzeigecharakteristika eine mit den Behandlungsplandaten verknüpfte Dosisintensität repräsentiert.

5. Der Verfahren nach einem der vorhergehenden Ansprüche, wobei für zumindest eine Teilmenge der Landmarken die Intensität zeitlich variabel mit einer vorgegebenen Frequenz ist, um die Visualisierung der Darstellung der anatomischen Strukturen, die zum 3D-Körpermodell gehört, insbesondere in dem Dosisverteilungsbereich entsprechenden Körperabschnitt des Subjekts zu erleichtern.

6. Der Verfahren nach einem der vorhergehenden Ansprüche, ferner umfassend, vor dem Erzeugen der o.g. Punktwolke, das Empfangen eines Punktdichteparameters, wobei das Erzeugen der Punktwolke auf Grundlage des genannten Punktdichteparameters erfolgt.

7. Der Verfahren nach einem der vorhergehenden Ansprüche, wobei die Punktwolke dynamisch ist und mindestens eine Teilmenge der Landmarken sich in der Umgebung ihrer Ausgangskoordinaten bewegt, um die Visualisierung der Darstellung der anatomischen Strukturen, die zum 3D-Körpermodell gehört, insbesondere in dem Dosisverteilungsbereich entsprechenden Körperabschnitt des Subjekts zu erleichtern.

8. Der Verfahren nach einem der vorhergehenden Ansprüche, wobei der Schritt des Anzeigens der dreidimensionalen Szene auf einer stereoskopischen Anzeigeeinheit implementiert ist, um die Wahrnehmung der Behandlungsplandaten im 3D-Raum mit verbesserter Tiefenwahrnehmung weiter zu erhöhen.

9. Der Verfahren nach einem der vorhergehenden Ansprüche, ferner umfassend das Empfangen mindestens eines Interessen-3D-Modells einer interessierenden Struktur des Subjekts, wobei für eine erste Teilmenge der Landmarken mindestens ein Anzeigecharakteristikum einem ersten Wert zugeordnet ist und für eine zweite Teilmenge der Landmarken das genannte mindestens eine Anzeigecharakteristikum einem zweiten Wert zugeordnet ist, wobei sich der zweite Wert vom ersten Wert unterscheidet, und wobei sich die erste Teilmenge der Landmarken innerhalb des mindestens einen Interessen-3D-Modells befindet und die zweite Teilmenge der Landmarken außerhalb des Interessen-3D-Modells.

10. Der Verfahren nach Anspruch 9, wobei die interessierende Struktur ein Risikoorgan repräsentiert.

11. Der Verfahren nach Anspruch 9, wobei die interessierende Struktur eine Zielzone ist, wobei die Zielzone vorzugsweise mindestens eine der folgenden Strukturen repräsentiert makroskopisches Tumorvolumen, klinisches Zielvolumen oder geplantes Zielvolumen.

12. Der Verfahren nach einem der vorhergehenden Ansprüche, ferner umfassend die Schritte:
- Empfangen zweiter Behandlungsplandaten, die eine zweite 3D-Verteilung einer zweiten Behandlungsdosis repräsentieren, welche während einer Strahlentherapie, einer Ablation, einer laserbasierten oder einer ultraschallbasierten Behandlung in den genannten Bereich des Körpers des Subjekts verabreicht werden soll,
- Erzeugen einer zweiten Punktwolke auf Grundlage der o.g. zweiten Behandlungsplandaten, wobei jedem Punkt der zweiten Punktwolke eine Landmarke zugeordnet ist, die Anzeigecharakteristika aufweist, welche mindestens eine Form, eine Größe und eine Intensität umfassen, wobei die Anzeigecharakteristika den entsprechenden zweiten Behandlungsplandaten zugeordnet sind,
- Anzeigen einer Überlagerung der dreidimensionalen Szene und der genannten zweiten Punktwolke.

13. Der Verfahren nach einem der vorhergehenden Ansprüche, wobei das genannte 3D-Körpermodell basierend auf einer Vielzahl von Bildern erzeugt wird, die den o.g. zumindest teilweisen Körperabschnitt des Subjekts umfassen, wobei die Bilder CT-Aufnahmen, MRT-Aufnahmen oder Cone-Beam-CT-Aufnahmen sind.

14. Der Verfahren nach einem der vorhergehenden Ansprüche, ferner umfassend die Schritte:
- Berechnen mindestens einer 2D-Projektion der genannten dreidimensionalen Szene auf mindestens eine 2D-Projektionsebene,
- Anzeigen der genannten mindestens einen 2D-Projektion.

15. Der Verfahren nach einem der vorhergehenden Ansprüche, wobei die Behandlungsplandaten Informationen über eine Art der Technologie, eine Art des Geräts, eine Anzahl von Geräten, eine Anzahl von Strahlen oder Parameter umfassen, die in den Geräteeinstellungen zur Erzeugung der genannten Behandlungsplandaten verwendet wurden.

## Revendications

1. Méthode mise en œuvre par ordinateur pour le guidage médical d'un utilisateur dans la validation d'une distribution de dose de traitement fournie par un système de planification de traitement, comprenant:
- recevoir (100) un modèle 3D du corps représentant au moins une portion du corps d'un sujet,
- recevoir (200) des données de plan de traitement représentant une distribution 3D d'une dose de traitement devant être délivrée à une région du corps du sujet lors d'une radiothérapie, d'une ablation, d'un traitement par laser ou par ultrasons,
- générer (300) un nuage de points basé sur au moins un sous-ensemble desdites données de plan de traitement, chaque point étant associé à un repère présentant des caractéristiques d'affichage comprenant au moins une forme, une taille, une intensité, une transparence ou une orientation, ces caractéristiques d'affichage étant associées aux données correspondantes du plan de traitement,
- afficher (400) une scène tridimensionnelle comprenant une superposition du modèle 3D du corps et dudit nuage de points,
**caractérisé en ce que** les caractéristiques d'affichage des repères associés auxdites données de plan de traitement dans différents plans de profondeur de la scène tridimensionnelle sont distinctes, de sorte que ces repères sont mieux perçus par rapport à la représentation classique de la dose sous forme de superpositions colorées continues.

2. Méthode selon la revendication 1, dans laquelle l'étape de génération (300) du nuage de points comprend :
a) créer un maillage à partir desdites données de plan de traitement, ledit maillage comprenant une pluralité de sommets, et dans laquelle, pour au moins un sous-ensemble de sommets, chaque sommet correspond à un point du nuage de points.

3. Méthode selon la revendication 2, comprenant en outre les étapes de :
b) appliquer un décalage de coordonnées aléatoire à chaque sommet, ledit décalage de coordonnées aléatoire étant inférieur à la distance entre deux sommets voisins, pour obtenir des points décalés, et
c) associer à chaque point décalé un repère, l'étape d'affichage (400) de la scène tridimensionnelle comprenant l'affichage des repères aux coordonnées des points décalés.

4. Méthode selon l'une quelconque des revendications précédentes, dans laquelle l'une des caractéristiques d'affichage est représentative de l'intensité de la dose associée aux données du plan de traitement.

5. Méthode selon l'une quelconque des revendications précédentes, dans laquelle pour au moins un sous-ensemble des repères, l'intensité est variable dans le temps selon une fréquence prédéterminée, afin de faciliter la visualisation desdites représentations des structures anatomiques appartenant au modèle 3D du corps, notamment dans la région du corps du sujet correspondant à la distribution de la dose de traitement.

6. Méthode selon l'une quelconque des revendications précédentes, comprenant en outre, avant la génération dudit nuage de points, la réception d'un paramètre de densité de points, et dans laquelle l'étape de génération du nuage de points est basée sur ledit paramètre de densité de points.

7. Méthode selon l'une quelconque des revendications précédentes, dans laquelle le nuage de points est dynamique et au moins un sous-ensemble des repères se déplace à proximité de ses coordonnées initiales, afin de faciliter la visualisation desdites représentations des structures anatomiques appartenant au modèle 3D du corps, notamment dans la région du corps du sujet correspondant à la distribution de la dose de traitement.

8. Méthode selon l'une quelconque des revendications précédentes, dans laquelle l'étape d'affichage de la scène tridimensionnelle est implémentée sur une unité d'affichage stéréoscopique, afin d'augmenter encore la perception des données du plan de traitement dans l'espace 3D avec une perception améliorée de la profondeur.

9. Méthode selon l'une quelconque des revendications précédentes, comprenant en outre la réception d'au moins un modèle 3D d'une structure d'intérêt du sujet, et dans laquelle pour un premier sous-ensemble des repères, au moins une caractéristique d'affichage est assignée à une première valeur, et pour un second sous-ensemble des repères, ladite caractéristique d'affichage est assignée à une seconde valeur, ladite seconde valeur étant différente de la première valeur, le premier sous-ensemble des repères étant situé à l'intérieur du ou des modèles 3D d'intérêt, et le second sous-ensemble des repères étant situé à l'extérieur du ou des modèles 3D d'intérêt.

10. Méthode selon la revendication 9, dans laquelle la structure d'intérêt est représentative d'un organe à risque.

11. Méthode selon la revendication 9, dans laquelle la structure d'intérêt est une zone cible, de préférence la zone cible est représentative d'au moins un parmi le volume de tumeur brute, le volume cible clinique ou le volume cible de planification.

12. Méthode selon l'une quelconque des revendications précédentes, comprenant en outre les étapes de :
- recevoir des données de second plan de traitement représentant une seconde distribution 3D d'une seconde dose de traitement, devant être délivrée à ladite région du corps du sujet lors d'une radiothérapie, d'une ablation, d'un traitement par laser ou par ultrasons,
- générer un second nuage de points basé sur lesdites données du second plan de traitement, chaque point du second nuage de points étant associé à un repère ayant des caractéristiques d'affichage comprenant au moins une forme, une taille et une intensité, ces caractéristiques d'affichage étant associées aux données correspondantes du second plan de traitement,
- afficher une superposition de la scène tridimensionnelle et dudit second nuage de points.

13. Méthode selon l'une quelconque des revendications précédentes, dans laquelle ledit modèle 3D du corps est obtenu à partir d'une pluralité d'images comprenant ladite portion du corps du sujet, ces images étant des images de tomodensitométrie (CT), des images de résonance magnétique (IRM), ou des images de tomodensitométrie à faisceau conique (cone beam CT).

14. Méthode selon l'une quelconque des revendications précédentes, comprenant en outre les étapes de :
- calculer au moins une projection 2D de ladite scène tridimensionnelle sur au moins un plan de projection 2D ;
- afficher ladite projection 2D.

15. Méthode selon l'une quelconque des revendications précédentes, dans laquelle les données du plan de traitement incluent des informations relatives à un type de technologie, un type de dispositif, un nombre de dispositifs, un nombre de faisceaux, ou des paramètres utilisés dans les réglages des dispositifs pour obtenir lesdites données du plan de traitement.
